# EUROPEAN PATENT SPECIFICATION

(11) **EP 2 305 263 B1**
(45) Date of publication and mention of the grant of the patent: **19.09.2012**
(21) Application number: 10178116.9
(22) Date of filing: 07.06.2007
(51) Int. Cl.: A61K 31/506, A61K 9/16, A61P 35/02

(54) **Stabilized amorphous forms of imatinib mesylate**
Stabilisierte amorphe Formen von Imatinib Mesylat
Formes amorphes stabilisées du mésylate de l'imatinib

(30) Priority: 07.06.2007 EP 07109816
(43) Date of publication of application: 06.04.2011
(62) Divisional of application: 07109816.4
(73) Proprietor: Novartis AG, 4056 Basel (CH)
(72) Inventor: Goncalves, Elisabete, 4055 Basel (CH); Kalb, Oskar, 4054 Basel (CH); Mutz, Michael, 79104 Freiburg (DE); Wirth, Wolfgang, 4422 Arisdorf (CH)
(74) Representative: Roth, Peter Richard

(56) References cited:
- WO-A-2004/106326
- WO-A-2006/121941
- US-A1- 2006 275 372
- BENI ET AL: "Cyclodextrin/imatinib complexation: Binding mode and charge dependent stabilities", EUROPEAN JOURNAL OF PHARMACEUTICAL SCIENCES, ELSEVIER, AMSTERDAM, NL, vol. 30, no. 2, 26 January 2007 (2007-01-26), pages 167-174, XP005738249, ISSN: 0928-0987
- LEUNER C ET AL: "Improving drug solubility for oral delivery using solid dispersions", EUROPEAN JOURNAL OF PHARMACEUTICS AND BIOPHARMACEUTICS, ELSEVIER SCIENCE PUBLISHERS B.V., AMSTERDAM, NL, vol. 50, no. 1, 3 July 2000 (2000-07-03), pages 47-60, XP004257179, ISSN: 0939-6411
- DOELKER E: "MODIFICATIONS CRISTALLINES ET TRANSFORMATIONS POLYMORPHES AU COURS DES OPERATIONS GALENIQUES CRYSTALLINE MODIFICATIONS AND POLYMORPHISM CHANGES DURING DRUG MANUFACTURE", ANNALES PHARMACEUTIQUES FRANCAISES, MASSON, PARIS, FR, vol. 60, no. 3, 2002, pages 161-176, XP009003219, ISSN: 0003-4509

## Description

The invention relates to pharmaceutical compositions comprising a formulation principle that stabilizes the amorphous form of Imatinib mesylate and amorphous Imatinib mesylate, wherein the formulation principle is selected from (a) certain solid dispersions as further described below and (b) dry co-milling with certain excipients as also further described below; to the use of such pharmaceutical compositions for the therapeutic treatment of warm-blooded animals, especially humans, and to the use of these formulation principles stabilizing the amorphous form of Imatinib mesylate as an intermediate for the preparation of pharmaceutical compositions.

### Background to the invention

It is well known that molecules can arrange to form different crystal polymorphs. Polymorphs have the same composition, but exhibit different solid-state properties as e.g. stability or solubility.

The preparation of 4-(4-methylpiperazin-1-ylmethyl)-N-[4-methyl-3-(4-(pyridin-3-yl)-pyrimidin-2-ylamino-)phenyl]-benzamide, also known as Imatinib, and its use, especially as an anti-tumour agent, are described in Example 21 of US 5,521,184. The compound is exemplified in these publications only in free form (not as a salt).

4-(4-Methylpiperazin-1-ylmethyl)-N-[4-methyl-3-(4-(pyridin-3-yl)-pyrimidin-2-ylamino)-phenyl]-benzamide mesylate, also known as Imatinib mesylate or STI571, as well as the alpha and the beta crystal form thereof are described in US 6,894,051. Imatinib mesylate is the active ingredient of the drug Gleevec® (Glivec®) which is an approved medicament for the treatment of Chronic Myeloid Leukemia (CML) and gastrointestinal stromal tumors (GIST) as well as a number of rare proliferative disorders.

In US 6,894,051 it is reported that the beta crystal modification of Imatinib mesylate is thermodynamically more stable than the alpha form at temperatures below 140 °C. Furthermore, both forms are thermodynamically more stable than the amorphous form of Imatinib mesylate. The different stabilities have the effect that the meta-stable amorphous form of Imatinib mesylate bears the risk of conversion into a more stable modification such as the alpha or the beta crystal form.

It was now surprisingly found that the amorphous form of Imatinib mesylate can be stabilized by various formulation principles. Hence, in a first aspect the present invention relates to the use of formulation principles that stabilize the amorphous form of Imatinib mesylate as an intermediate for the preparation of a pharmaceutical composition comprising the amorphous form of Imatinib mesylate, as well as pharmaceutical compositions comprising such a formulation principle and amorphous Imatinib mesylate. More specifically, the present invention describes pharmaceutical compositions comprising formulation principles capable of stabilizing the amorphous form of Imatinib mesylate and amorphous Imatinib mesylate, optionally together with at least one pharmaceutically acceptable carrier, wherein the formulation principle is selected from (a) solid dispersions, wherein the solid dispersion comprises at least one further excipient, which is selected from cellulose derivatives, polyvinylpyrrolidone, polyethyleneglycols of various molecular weights, polyethylene-/polypropylene-/polyethylene-oxide block copolymers and polymethacrylates, and (b) dry co-milling with excipients selected from polyvinylpyrrolidone, cellulose derivatives, earth alkali metal silicas and silicates.

The term "stabilizing Imatinib mesylate in the amorphous form" as used herein means especially that Imatinib mesylate is maintained in the amorphous form after 1 month of storage at 40°C at 75% relative humidity. The term "Imatinib mesylate in the amorphous form" designates a modification of Imatinib mesylate, which does not show any reflexes in X-ray diagrams that would correlate to reflexes observed for a crystalline modification of Imatinib mesylate , especially the alpha or beta crystal form of Imatinib mesylate.

Compared to crystalline forms of Imatinib mesylate, a stabilized amorphous form of Imatinib mesylate offers a number of advantages including economic advantages and a higher dissolution rate.

Crystalline material is generally obtained through a crystallization process, which constitutes an additional manufacturing step. In particular, complete crystallization from a mother liquor is often a time consuming process step, thus binding production capacity. Therefore, a stabilized amorphous form of Imatinib mesylate is an attractive alternative to crystalline forms under economic aspects.

In general, amorphous forms of a substance show a higher dissolution rate than crystalline forms of the same substance. Furthermore, a high dissolution rate can result in oversaturated solutions. The higher dissolution rate as well as the potentially obtained oversaturated solution can also result in better bioavailability of the amorphous form of a substance compared to a crystalline form thereof. The same amount of drug could thus be absorbed by a patient obtaining a lower dose of a given drug. This lowers the risk of local side effects in the patients caused by not absorbed materials and also has a cost saving effect.

### Detailed description of the invention

The formulation principles described herein can be used as starting materials for the manufacture of pharmaceutical compositions, such as tablets, suspensions, powders, sachets, capsules or suppositories, comprising amorphous Imatinib mesylate. Depending on the specific composition used, these compositions can, e.g., be applied oral, rectal, vaginal or by inhalation.

### 1. Solid dispersions

The solid dispersions of the present invention comprise amorphous Imatinib mesylate and at least one further excipient selected from cellulose derivatives, polyvinylpyrrolidone, polyethyleneglycols of various molecular weights, polyethylene-/polypropylene-/polyethyleneoxide block copolymers and polymethacrylates. Representative examples of cellulose derivatives include hydroxypropylmethylcellulose (HPMC), hydroxypropylcellulose (HPC), methylcellulose (MC), cellulose acetate phthalate (CAP), hydroxypropylmethylcellulose phthalate (HPMC-P), hydroxylpropyl methylcellulose acetate succinate (HPMC-AS), carboxymethylethylcellulose (CMEC). Other suitable excipients in solid dispersion formulations include, but are not limited to, polyvinylalcohol (PVA) and co-polymers thereof with PVP or with other polymers, polyacrylates, urea, chitosan and chitosan glutamate, sorbitol or other polyols such as mannitol.

Optionally, the solid dispersions comprise additionally at least one surfactant such as sodium dodecyl sulfate (SDS), polyoxyethylene sorbitan fatty acid esters such as Tween® 80, bile salts such as sodium deoxycholate, polyoxyethylene mono esters of a saturated fatty acid such as Solutol® HS 15, water soluble tocopheryl polyethylene glycol succinic acid esters such as Vitamin E TPGS.

The solid dispersions may contain amorphous Imatinib Mesylate in an amount by weight of the composition of about 0.01 % to about 80%; for example, in an amount by weight of about 0.01% to about 80%, 0.1% to about 70%, such as 1% to 60%, for example 2%, 5%, 10%, 20%, 30%, 40%, 50%, or 60%. The polymeric excipient may be present in an amount from about 0.1 % to 99.99% by weight of the composition. When a surfactant is present, it may generally be present in an amount of from about 0.01 % to about 30%, for example from about 1 % to about 20% by weight, e.g. 1 % to 15% by weight such as 5% to 15% by weight of the composition.

In one embodiment of the invention, the cellulose derivative is selected from hydroxypropylcellulose (HPC), hydroxypropylmethylcellulose (HPMC) and hydroxypropylmethylcellulose acetate succinate (HPMC-AS). The solid dispersion in such case comprises preferably between 50 and 90 % by weight of the cellulose derivative and 10 to 50 % by weight of amorphous Imatinib mesylate.

If polyvinylpyrrolidone is employed as further excipient in the solid dispersion, the solid dispersion preferably comprises between 50 and 90 % by weight of polyvinylpyrrolidone and 10 to 50 % by weight of amorphous Imatinib mesylate.

Suitable polyethyleneglycols are especially Polyethyleneglycol 8000 and Polyethyleneglycol 6000. The solid dispersion preferably comprises between 50 and 90 % by weight of a polyethyleneglycol and 10 to 50 % by weight of amorphous Imatinib mesylate.

A suitable polyethylene-/polypropylene-/polyethylene-oxide block copolymer is in particular Pluronic F68. The solid dispersion preferably comprises between 50 and 90 % by weight of a polyethylene-/polypropylene-/polyethylene-oxide block copolymer and 10 to 50 % by weight of amorphous Imatinib mesylate.

Eudragit^{®}L-100-55 and Eudragit^{®}E-100 are suitable polymethacrylates for the present invention. The solid dispersion preferably comprises between 50 and 90 % by weight of a polymethacrylates and 10 to 50 % by weight of amorphous Imatinib mesylate.

Optionally, surfactants can be added to solid dispersion. In such case, typically 1 to 10% by weight, preferably 2 to 4 % by weight, of the excipient mentioned above is replaced by a surfactant.

In one preferred embodiment of the invention, the solid dispersion compositions are prepared by melt extrusion.

### 2. Co-milling with excipients

In one embodiment of the present invention, the formulation principle for stabilizing amorphous Imatinib mesylate is co-milling with selected excipients. In such embodiment, amorphous Imatinib mesylate can be dry co-milled or wet co-milled with the added excipients.

For dry co-milling the added excipient can be selected from polyvinylpyrrolidone, e.g. PVPK30, cellulose derivatives, such as, but not limited to, hydroxypropylcellulose (HPC), hydroxypropylmethylcellulose (HPMC), hydroxypropylmethylcellulose acetate succinate (HPMC-AS), hydroxypropylcellulose phthalate (HPMC-P), methylcellulose (MC), and earth alkali metal silicas and silicates, e.g. fumed silicas, precipitated silicas, calcium silicates, such as Zeopharm^{®}600, or magnesium aluminometasilicates such as Neusilin US2.

Formulation principles obtained by dry co-milling preferably comprise between 10 to 50 %, more preferably 30 to 50 %, by weight of amorphous Imatinib mesylate.

### 3. Methods of using the stabilized amorphous form of Imatinib mesylate

Stabilized amorphous forms of Imatinib mesylate possess valuable pharmacological properties and may, for example, be used as an anti-tumour agent or as an agent to treat restenosis.

The present invention relates especially to the use of a pharmaceutical composition as described herein comprising the stabilized amorphous form of Imatinib mesylate in the treatment of one of the diseases mentioned herein.

The antiproliferative, especially anti-tumour, activity of the methanesulfonic acid addition salt of a compound of formula I in vivo is, for example, described for the treatment of abl-dependent tumours in Nature Med. 2, 561-6 (1996).

The invention relates moreover to the use of a pharmaceutical composition as described herein for use of the treatment of a disease selected from metastatic, inoperable GIST, advanced chronic myeloid leukemia, newly diagnosed chronic myeloid leukemia, pediatric Philadelphia chromosome-positive chronic myeloid leukemia, Philadelphia chromosome-positive acute lymphocytic leukemia (ALL), glioblastoma multiforme, dermatofibrosarcoma protuberans (DFSP), hypereosinophilic sindrome (HES), and chronic myelomonocytic leucemia (CMML).

In preferred embodiments, the present invention relates to the use in of a stabilized amorphous form of Imatinib mesylate in the treatment of one of the disorders listed below:
1. GIST,
2. advanced chronic myeloid leukemia,
3. newly diagnosed chronic myeloid leukemia,
4. pediatric Philadelphia chromosome-positive chronic myeloid leukemia,
5. Philadelphia chromosome-positive acute lymphocytic leukemia (ALL),
6. glioblastoma multiforme, preferably in combination with hydroxyurea,
7. dermatofibrosarcoma protuberans (DFSP),
8. hypereosinophilic sindrome (HES),
9. chronic myelomonocytic leucemia (CMML), and
10. idiopathic pulmonary fibrosis.

Depending on species, age, individual condition, mode of administration, and the clinical picture in question, effective doses, for example daily doses of a stabilized amorphous form of Imatinib mesylate, which correspond to about 100-2000 mg, preferably 200-1000 mg, especially 250-800 mg of Imatinib mesylate, are administered to warm-blooded animals of about 70 kg bodyweight. Preferably, daily dosages of a stabilized amorphous form of Imatinib mesylate, which correspond to about 400 mg or 600 mg of Imatinib mesylate, are administered orally once daily, preferably together with a meal and a large glass of water (about 200 mL). Daily doses of a stabilized amorphous form of Imatinib mesylate, which correspond to about 800 mg of Imatinib mesylate are preferably administered in the form of 400 mg dosages twice daily together with food.

In one embodiment, the formulation principle is provided in the form of a capsule, which is a hard gelatine capsule containing a dry powder blend. The capsule shell preferably contains gelatine and titanium dioxide as well as red iron oxide. The ratio of weight of capsule fill to capsule shell is preferably between about 100:25 and 100:50, more preferably between 100:30 and 100:40.

Accordingly, the present invention provides
(a) pharmaceutical compositions comprising a formulation principle that stabilizes the amorphous form of Imatinib mesylate and amorphous Imatinib mesylate, optionally together with at least one pharmaceutically acceptable excipient, especially wherein the formulation principle is selected from (i) solid dispersions, wherein the solid dispersion comprises at least one further excipient, which is selected from cellulose derivatives, polyvinylpyrrolidone, polyethyleneglycols of various molecular weights, polyethylene-/polypropylene-/polyethylene-oxide block copolymers and polymethacrylates; and (ii) dry co-milling with excipients selected from polyvinylpyrrolidone, cellulose derivatives, earth alkali metal silicas and silicates;
(b) a capsule comprising a formulation principle as defined above that stabilizes the amorphous form of Imatinib mesylate and amorphous Imatinib mesylate, optionally together with at least one pharmaceutically acceptable excipient, which contains between an amount of stabilized amorphous Imatinib mesylate which corresponds to 50 mg and 200 mg of Imatinib mesylate, and, optionally wherein the shell contains gelatine and/or, wherein the shell contains titanium dioxide and/or wherein the shell contains red iron oxide. In such a capsule, the ratio of weight of capsule fill to capsule shell is between about 100:25 and 100:50, especially between 100:30 and 100:40;
(c) a tablet comprising a formulation principle as defined above that stabilizes the amorphous form of Imatinib mesylate and amorphous Imatinib mesylate, optionally together with at least one pharmaceutically acceptable excipient, in particular comprising an amount of stabilized amorphous Imatinib mesylate which corresponds to 100 mg, 400 mg or 800 mg of Imatinib mesylate;
(d) a pharmaceutical composition as described above for the use of the treatment of a disease selected from metastatic, inoperable GIST, advanced chronic myeloid leukemia, newly diagnosed chronic myeloid leukemia, pediatric Philadelphia chromosome-positive chronic myeloid leukemia, Philadelphia chromosome-positive acute lymphocytic leukemia (ALL), glioblastoma multiforme, dermatofibrosarcoma protuberans (DFSP), hypereosinophilic sindrome (HES), and chronic myelomonocytic leucemia (CMML);
(e) the use of a formulation principle as defined above which stabilizes the amorphous form of Imatinib mesylate as an intermediate for the preparation of a pharmaceutical composition comprising the amorphous form of Imatinib mesylate.

The following Examples illustrate the invention without limiting the scope thereof. The examples listed below describe formulations where no crystalline drug is detected after 1 mo. storage at 40°C/75% RH.

### EXAMPLE 1

This Example lists representative solid dispersion compositions of amorphous Imatinib Mesylate (Table 1) and describes making a solid dispersion according to the invention. Imatinib Mesylate (crystal form beta) is formulated as a solid dispersion using high throughput screening technology (HTS) as follows. A quantity of Imatinib Mesylate is first dissolved in a suitable solvent (95% ethanol or acetone:ethanol:water (50:40:10) to provide a stock solution (25 mg/mL). An adequate volume of this solution (40 to 200 µL) is then dispensed into each well of a 96-well plate HTS Crissy Platform to deliver the desired amount of Imatinib Mesylate (1 to 5 mg) to each well. The solvent is then evaporated to dryness. A quantity of each excipient is dissolved or suspended in a suitable solvent (95% ethanol or acetone:ethanol:water (50:40:10)) to provide a stock solution (25 mg/mL). An adequate volume of the excipient stock solution (40 to 360 µL) is then added to each well containing a quantity of Imatinib Mesylate. Contents of each well are mixed and the solvent is evaporated to dryness. The 96-well plate is scanned using X-ray powder diffraction (XRPD) to monitor the presence of crystalline Imatinib Mesylate both prior and after storage for 1 mo. at 40°C/75% RH.

**Table 1 - Representative solid dispersion compositions of amorphous Imatinib Mesylate prepared by the solvent evaporation method**

| Imatinib Mesylate (wt%) | Excipient type | Excipient (wt%) | Solvent composition |
|---|---|---|---|
| **Cellulose derivatives** | | | |
| 10 | hydroxypropylcellulose (HPC) | 90 | 95% Ethanol |
| 50 | HPC | 50 | 95% Ethanol |
| 10 | hydroxypropylmethylcellulose (HPMC) | 90 | Acetone:ethanol: water (50:40:10) |
| 50 | HPMC | 50 | Acetone:ethanol: water (50:40:10) |
| 10 | hydroxypropylmethylcellulose acetate succinate (HPMC-AS) | 90 | 95% Ethanol |
| 50 | HPMC-AS | 50 | 95% Ethanol |

| **Polyvinylpyrrolidone** | | | |
|---|---|---|---|
| 10 | Polyvinylpyrrolidone (PVPK30) | 90 | 95% Ethanol |
| 50 | PVPK30 | 50 | 95% Ethanol |

| **Polyethyleneglycols** | | | |
|---|---|---|---|
| 10 | Polyethyleneglycol 6000 (PEG6000) | 90 | 95% Ethanol |
| 50 | PEG6000 | 50 | 95% Ethanol |
| 10 | Polyethyleneglycol 8000 (PEG8000) | 90 | Acetone:ethanol: water (50:40:10) |
| 50 | PEG8000 | 50 | 95% Ethanol |

| **Polyethylene-/polypropylene-/polyethylene-oxide block copolymers** | | | |
|---|---|---|---|
| 10 | Pluronic F68 | 90 | 95% Ethanol |
| 30 | Pluronic F68 | 70 | 95% Ethanol |
| 50 | Pluronic F68 | 50 | Acetone:ethanol: water (50:40:10) |

| **Polymethacrylates** | | | |
|---|---|---|---|
| 10 | Eudragit^{®}E-100 | 90 | 95% Ethanol |
| 30 | Eudragit^{®}E-100 | 70 | 95% Ethanol |
| 10 | Eudragit^{®}L-100-55 | 90 | 95% Ethanol |
| 30 | Eudragit^{®}L-100-55 | 70 | 95% Ethanol |

| **Compositions comprising polymers of each class and one surfactant** | | | |
|---|---|---|---|
| 10 | HPC / vitamin E TPGS | 48 / 2 | 95% Ethanol |
| 10 | HPC / Solutol HS 15 | 48 / 2 | 95% Ethanol |
| 10 | HPMC / vitamin E TPGS | 48 / 2 | 95% Ethanol |
| 10 | HPMC / Solutol HS 15 | 48 / 2 | 95% Ethanol |
| 10 | HPMC-AS / vitamin E TPGS | 48 / 2 | 95% Ethanol |
| 10 | HPMC-AS / Solutol HS 15 | 48 / 2 | 95% Ethanol |
| 10 | PVPK30 / vitamin E TPGS | 48 / 2 | 95% Ethanol |
| 10 | PVPK30 / Solutol HS 15 | 48 / 2 | 95% Ethanol |
| 10 | PEG6000 / vitamin E TPGS | 48 / 2 | 95% Ethanol |
| 10 | PEG8000 / vitamin E TPGS | 48 / 2 | 95% Ethanol |
| 10 | Pluronic F68 / vitamin E TPGS | 48 / 2 | 95% Ethanol |
| 10 | Pluronic F68 / Solutol HS 15 | 48 / 2 | 95% Ethanol |
| 10 | Eudragit^{®}E-100 / vitamin E TPGS | 48 / 2 | 95% Ethanol |
| 10 | Eudragit^{®}E-100 / Solutol HS 15 | 48 / 2 | 95% Ethanol |
| 10 | Eudragit^{®}L-100-55 / vitamin E TPGS | 48 / 2 | 95% Ethanol |
| 10 | Eudragit^{®}L-100-55 / Solutol HS 15 | 48 / 2 | 95% Ethanol |

### EXAMPLE 2

This Example lists representative dry co-milled Imatinib Mesylate - excipient compositions (Table 2) and illustrates making the composition according to the invention. Imatinib Mesylate (amorphous form) is co-milled with excipients in the dry state as follows. A blend (2.5 g total amount) of Imatinib Mesylate and excipient is mixed with zirconia beads (5 g, 3 mm in diameter) for 10 min in a bench-top turbula prior starting the milling experiment. An adequate amount of the Imatinib Mesylate/excipient/zirconia beads blend (3.75 g) is then transferred to the milling vessel of a vibration mill, and milled for 2 hours at ambient temperature and 1000 rpm. The final powder is analyzed by XRPD both prior and after storage for 1 mo. at 40°C/75% RH.

**Table 2 - Representative dry co-milled Imatinib Mesylate - excipients compositions**

| Imatinib Mesylate (wt%) | Excipient type | Excipient (wt%) |
|---|---|---|
| 30 | HPMC | 70 |
| 30 | PVPK30 | 70 |
| 50 | PVPK30 | 50 |
| 30 | Precipitated calcium silicate (Zeopharm^{®}600) | 70 |

### EXAMPLE 3

This Example lists representative solid dispersion compositions of amorphous Imatinib Mesylate (Table 3) prepared by melt extrusion, and illustrates making the composition according to the invention.

Imatinib Mesylate (amorphous form) is blended with excipients as follows. A blend comprising 50wt% of Imatinib Mesylate and 50wt% of excipient (listed in Table 3) is prepared in a mortar and pestle prior starting the extrusion experiment. The blend is then transferred to the extrusion vessel of a Haake mini lab extruder, and processed for x hours at 165°C and 170°C for Eudragit L100-55 and PVPK30, respectively. The final extrudate is powdered using a mortar and pestle and analyzed by XRPD and DSC both prior and after storage for 1 mo. and 4 mo. at 40°C/75% RH.

**Table 3 - Representative solid dispersion compositions of Imatinib Mesylate prepared by melt extrusion**

| Imatinib Mesylate (wt%) | Excipient type | Excipient (wt%) |
|---|---|---|
| 50 | PVPK30 | 50 |
| 50 | Eudragit L100-55 | 50 |

## Claims

1. A pharmaceutical composition comprising a formulation principle that stabilizes the amorphous form of Imatinib mesylate and amorphous Imatinib mesylate, optionally together with at least one pharmaceutically acceptable carrier, wherein the formulation principle is selected from (a) solid dispersions, wherein the solid dispersion comprises at least one further excipient, which is selected from cellulose derivatives, polyvinylpyrrolidone, polyethyleneglycols of various molecular weights, polyethylene-/polypropylene-/polyethylene-oxide block copolymers and polymethacrylates; and (b) dry co-milling with excipients selected from polyvinylpyrrolidone, cellulose derivatives, earth alkali metal silicas and silicates.

2. The pharmaceutical composition according to claim 1, wherein the formulation principle is a solid dispersion, wherein the solid dispersion comprises at least one further excipient, which is selected from cellulose derivatives, polyvinylpyrrolidone, polyethyleneglycols of various molecular weights, polyethylene-/polypropylene-/polyethylene-oxide block copolymers and polymethacrylates.

3. The pharmaceutical composition according to claim 1, wherein the formulation principle is dry co-milling with excipients selected from polyvinylpyrrolidone, cellulose derivatives, such as hydroxypropylcellulose (HPC), hydroxypropylmethylcellulose (HPMC), hydroxypropylmethylcellulose acetate succinate (HPMC-AS), hydroxypropylcellulose phthalate (HPMC-P), or methylcellulose (MC), and earth alkali metal silicas and silicates.

4. The pharmaceutical composition according to claim 1 for use of the treatment of a disease selected from metastatic, inoperable GIST, advanced chronic myeloid leukemia, newly diagnosed chronic myeloid leukemia, pediatric Philadelphia chromosome-positive chronic myeloid leukemia, Philadelphia chromosome-positive acute lymphocytic leukemia (ALL), glioblastoma multiforme, dermatofibrosarcoma protuberans (DFSP), hypereosinophilic sindrome (HES), and chronic myelomonocytic leucemia (CMML).

5. The use of a formulation principle which stabilizes the amorphous form of Imatinib mesylate as an intermediate for the preparation of a pharmaceutical composition comprising the amorphous form of Imatinib mesylate, wherein the formulation principle is selected from (a) solid dispersions wherein the solid dispersion comprises at least one further excipient, which is selected from cellulose derivatives, polyvinylpyrrolidone, polyethyleneglycols of various molecular weights, polyethylene-/polypropylene-/polyethylene-oxide block copolymers and polymethacrylates; and (b) dry co-milling with excipients selected from polyvinylpyrrolidone, cellulose derivatives, earth alkali metal silicas and silicates.

## Patentansprüche

1. Pharmazeutische Zusammensetzung, die ein Formulierungsprinzip, das die amorphe Form von Imatinib-mesylat stabilisiert, und amorphes Imatinib-mesylat, optional zusammen mit mindestens einem pharmazeutisch akzeptablen Träger umfasst, wobei das Formulierungsprinzip ausgewählt ist aus (a) festen Dispersionen, wobei die feste Dispersion mindestens einen weiteren Hilfsstoff umfasst, der aus Cellulosederivaten, Polyvinylpyrrolidon, Polyethylenglykolen verschiedener Molekulargewichte, Polyethylen-/Polypropylen-/Polyethylenoxid-Blockcopolymeren und Polymethacrylaten ausgewählt ist, und (b) einem trockenen Co-Vermahlen mit Hilfsstoffen, die aus Polyvinylpyrrolidon, Cellulosederivaten, Erdalkalimetall-Silciumdioxidverbindungen und -Silicaten ausgewählt sind.

2. Pharmazeutische Zusammensetzung nach Anspruch 1, wobei das Formulierungsprinzip eine feste Dispersion ist, wobei die feste Dispersion mindestens einen weiteren Hilfsstoff umfasst, der aus Cellulosederivaten, Polyvinylpyrrolidon, Polyethylenglykolen verschiedener Molekulargewichte, Polyethylen-/Polypropylen-/Polyethylenoxid-Blockcopolymeren und Polymethacrylaten ausgewählt ist.

3. Pharmazeutische Zusammensetzung nach Anspruch 1, wobei das Formulierungsprinzip ein trockenes Co-Vermahlen mit Hilfsstoffen, die aus Polyvinylpyrrolidon, Cellulosederivaten, wie Hydroxypropylcellulose (HPC), Hydroxypropylmethylcellulose (HPMC), Hydroxypropylmethylcellulose-acetatsuccinat (HPMC-AS), Hydroxypropylcellulose-phthalat (HPMC-P) oder Methylcellulose (MC) und Erdalkalimetall-Siliciumdioxidverbindungen und -Silicaten ausgewählt sind, umfasst.

4. Pharmazeutische Zusammensetzung nach Anspruch 1 zur Verwendung bei der Behandlung einer Erkrankung, die aus metastatischem, inoperablem GIST, fortgeschrittener chronischer myeloischer Leukämie, neu diagnostizierter chronischer myeloischer Leukämie, Philadelphia-Chromosom-positiver chronischer myeloischer Leukämie des Kindesalters, Philadelphia-Chromosom-positiver akuter lymphatischer Leukämie (ALL), Glioblastoma multiforme, Dermatofibrosarcoma protuberans (DFSP), Hypereosinophilie-Syndrom (HES) und chronischer myelomonozytärer Leukämie (CMML) ausgewählt ist.

5. Verwendung eines Formulierungsprinzips, das die amorphe Form von Imatinib-mesylat als ein Zwischenprodukt für die Herstellung einer pharmazeutischen Zusammensetzung stabilisiert, die die amorphe Form von Imatinib-mesylat umfasst, wobei das Formulierungsprinzip ausgewählt ist aus (a) festen Dispersionen, wobei die feste Dispersion mindestens einen weiteren Hilfsstoff umfasst, der aus Cellulosederivaten, Polyvinylpyrrolidon, Polyethylenglykolen verschiedener Molekulargewichte, Polyethylen-/Polypropylen-/Polyethylenoxid-Blockcopolymeren und Polymethacrylaten ausgewählt ist, und (b) einem trockenen Co-Vermahlen mit Hilfsstoffen, die aus Polyvinylpyrrolidon, Cellulosederivaten, Erdalkalimetall-Silciumdioxidverbindungen und -Silicaten ausgewählt sind.

## Revendications

1. Composition pharmaceutique comprenant un principe de formulation qui stabilise la forme amorphe du mésylate d'Imatinib et le mésylate d'Imatinib amorphe, avec éventuellement au moins un véhicule acceptable sur le plan pharmaceutique, dans laquelle le principe de formulation est choisi parmi (a) des dispersions solides, où la dispersion solide comprend au moins un excipient supplémentaire, qui est choisi parmi les dérivés de cellulose, la polyvinylpyrrolidone, les polyéthylèneglycols de différents poids moléculaires, les copolymères séquencés de polyéthylène-/polypropylène-/oxyde de polyéthylène et les polyméthacrylates; et (b) un co-broyage à sec avec des excipients choisis parmi la polyvinylpyrrolidone, les dérivés de cellulose, les silices et silicates de métaux alcalino-terreux.

2. Composition pharmaceutique selon la revendication 1, dans laquelle le principe de formulation est une dispersion solide, où la dispersion solide comprend au moins un excipient supplémentaire, qui est choisi parmi les dérivés de cellulose, la polyvinylpyrrolidone, les polyéthylèneglycols de différents poids moléculaires, les copolymères séquencés de polyéthylène-/polypropylène-/oxyde de polyéthylène et les polyméthacrylates.

3. Composition pharmaceutique selon la revendication 1, dans laquelle le principe de formulation est un co-broyage à sec avec des excipients choisis parmi la polyvinylpyrrolidone, les dérivés de cellulose, tels que l'hydroxypropylcellulose (HPC), l'hydroxypropylméthylcellulose (HPMC), l'acéto-succinate d'hydroxypropylméthylcellulose (HPMC-AS), le phtalate d'hydroxypropylcellulose (HPMC-P), ou la méthylcellulose (MC), et les silices et silicates de métaux alcalino-terreux.

4. Composition pharmaceutique selon la revendication 1 à utiliser pour le traitement d'une maladie choisie parmi les GIST métastatiques, inopérables, la leucémie myéloïde chronique avancée, une leucémie myéloïde chronique nouvellement diagnostiquée, la leucémie myéloïde chronique pédiatrique à chromosome Philadelphie positif, la leucémie aiguë lymphoblastique (LAL) à chromosome Philadelphie positif, le glioblastome multiforme, le dermato-fibrome de Darier-Ferrand (DFSP), le syndrome hyperéosinophilique (SHE) et la leucémie myélomonocytaire chronique (LMMC).

5. Utilisation d'un principe de formulation qui stabilise la forme amorphe du mésylate d'Imatinib comme intermédiaire pour la préparation d'une composition pharmaceutique comprenant la forme amorphe du mésylate d'Imatinib, ledit principe de formulation étant choisi parmi (a) des dispersions solides, où la dispersion solide comprend au moins un excipient supplémentaire, qui est choisi parmi les dérivés de cellulose, la polyvinylpyrrolidone, les polyéthylèneglycols de différents poids moléculaires, les copolymères séquencés de polyéthylène-/polypropylène-/oxyde de polyéthylène et les polyméthacrylates; et (b) un co-broyage à sec avec des excipients choisis parmi la polyvinylpyrrolidone, les dérivés de cellulose, les silices et silicates de métaux alcalino-terreux.
